# EUROPEAN PATENT APPLICATION

(11) **EP 2 186 411 A1**
(43) Date of publication of application: **19.05.2010**
(21) Application number: 08164494.0
(22) Date of filing: 17.09.2008
(51) Int. Cl.: A01N 59/16, A01N 59/00, A01N 37/44, A01N 33/24, A01N 33/08, A01N 37/52, A01N 33/04

(54) **Antimicrobial composition**

(71) Applicant: Taminco, 9000 Gent (BE)
(72) Inventor: Rabasse, Jean-Michel, 75008 Paris (FR); De Saegher, Johan, 9070 Destelbergen (BE); Demuynck, Marc, 9032 Wondelgem (BE); Roose, Peter, 9831 Sint-Martens-Latem (BE)
(74) Representative: Van Reet, Joseph

(57) **Abstract**

The invention relates to new antimicrobial compositions comprising a combination of
- at least one organic and / or inorganic peroxide,
- at least one silver source, and
- at least one specific nitrogen containing compound selected from N-methylated aminoacids, N-methylated aminoalcohols, N-methylated amine oxides, N-methylated amidines and poly amino hydrocarbon compounds, or
- at least one non glucogenic compatible solute.

## Description

The invention relates to the field of antimicrobial compositions, also referred to as antiseptics or disinfectants.

Ideally, an antiseptic or disinfectant should show a broad antimicrobial spectrum with a potent germicidal activity and a rapid activity onset combined with a long lasting effect. Such formulations should not be toxic to the host tissues and should not interfere with a healing or recovery effect in the infected host.
Studies with different biocides including silver compounds concluded that ionic silver kills a broad range of microbes at lower concentrations. Silver compounds are therefore used in clinical practices. In other studies the combined assessments of cellular cytotoxcicity and microbial activity showed that the silver compounds damage host cells more than they do to the test organisms. This toxic activity however depended strongly on the used medium. It is nevertheless important to reduce the stress of biocides on host tissues. Silver combined with peroxides should normally induce oxidative stress on the host. Therefore, it is important to introduce a stress reduction compound which does not alter the biocidal activity. Different silver salts, silver nanoparticles and silver compound containing devices have shown broad spectrum antimicrobial activity. Recently, it was even demonstrated that silver nanoparticles inhibit monkeypoxvirus replication at non-toxic concentrations. Silver salts also show interesting biological effects different from the antimicrobial effect on host tissues. It was also demonstrated that silver nitrate promoted plant regeneration from mature embryos in different wheat cultivars which confirmed the effect on somatic embryogenesis and plant regeneration in a number of dicotyledonous species of Brassica spp, pomegranate, cucumber, rice, maize, pear, millet and barley. Before, it was demonstrated that silver nitrate promoted shoot regeneration from callus cultures derived from immature embryos and that it inhibits the physiological action of ethylene by competing for its binding site, rather than effecting ethylene synthesis.
It is supposed that silver ions could interfere with polyamines.
Silver oxide has been incorporated in nanocomposites showing antibacterial activity. Different silver oxides carriers such as chitosan, alginates, proteins, polysaccharides, gums, celluloses and others have been used as reducing stabilizing agents. The mechanisms of the antimicrobial action of silver ions is supposed to be linked to the action on sulfhydryl groups. Addition of cysteine, or other compounds thiol groups neutralizes the silver ions action, but there is also an action on the hydrogen bounding and some essential enzymes causing the release of potassium ions from the bacteria. The bacterial cytoplasmic membrane has been reported to be the most essential target for silver. Silver ions have also been deposited in the vacuole and cell wall as granules, inhibiting cell division and provoking cell damage during the growth process. There is also supposed to exist an additional interaction with the DNA bases, inhibiting cell division and interfering with proton transport.
A synergistic effect between silver and copper ions was detected. Copper ions are omnipresent in different types of water. Silver nitrate is also used against warts.

Silver compounds have been used for thousand of years. They showed good results and beneficial effect in many diseases as hygienic compound, disinfectant, as compound to improve would healing, diabetic ulcers, etc...
The activity is most interesting to control microbial growth on living tissues and on food and feed products. The addition of silver compounds to water controls algae growth, development of pathogenic bacteria and fungi, biofilm formation in piping systems, on implants, catheters, rubbers, etc...
Nevertheless, silver resistant strains of microbes are evolving. It is therefore interesting combining silver compounds with a peroxide of a combination of peroxides to prevent resistant formation. The main advantage for using silver compounds is the persistent antimicrobial activity over a wide pH range. Silver compounds may even be used at very low concentrations in tap water to control microbial growth in combination with hydrogen peroxide. Since water is the major vehicle of disease transmission in all kind of organism, it is important to treat all kinds of water with the combination peroxide-silver. Another advantage of silver over other disinfectants is the remaining residual biocidal activity after the contact with the pathogens or organic material.

The combination of hydrogen peroxide and silver nitrate results in a synergy with a higher antimicrobial activity at a broader pH range compared to solely hydrogen peroxide. Not only hydrogen peroxide but also other organic or inorganic peroxides and even mixtures of different peroxides may be combined with silver compounds. The resulting antimicrobial activity is determined and different combinations are evaluated for the antimicrobial activity against different microbes in vitro. The silverperoxide combination is generally accepted as an ecologically friendly system for disinfection or decontamination and many researchers suggest using these formulations as a realistic alternative for chlorine containing antimicrobials. Silver compounds are very reactive and are finally precipitated as metallic silver, silver oxide or silver salts. Mostly, silver chloride is formed due to the omni presence of chloride ions in the environment and all kinds of process water and the extreme low solubility of this salt. Silver salts also show antimicrobial activity in the form of nanoparticles.

Peroxide and silver compounds primarily act on the microbial cytoplasmic membrane and hereby provoke oxidative stress. Enzymes are denatured and the membrane shows leakages with the release of cytoplasmic ingredients resulting in the death of the microbes. When such combinations are applied on "biological surfaces" (plant, leaves, roots, skin of animals or humans, fruits, vegetables, seeds, meet or fish products) oxidative stress occurs resulting in free radical initiated chain reactions causing deterioration of the biological material.

Against this complex background it has now been found surprisingly that a new antimicrobial composition can be proposed resulting in a higher biocidal activity at a broader pH range, based on the synergistic activity of a peroxide linked to a silver compound, where this synergistic effect is further supplemented with well defined compounds belonging to the class of nitrogen containing molecules and of so called "compatible solutes".
This combination has been found to result not only in an increased activity on bacteria due to the normalization of the cytoplasmatic turgor, but also in a better protection of host cells and host tissues against oxidative stress resulting from the use of peroxides and from other sources of biotic and abiotic stress.

The invention accordingly provides a new antimicrobial composition comprising a combination of
- at least one organic and / or inorganic peroxide,
- at least one silver source, and
- at least one specific nitrogen containing compound selected from N-methylated aminoacids, N-methylated aminoalcohols, N-methylated amine oxides, N-methylated amidines and poly amino hydrocarbon compounds, or
- at least one non glucogenic compatible solute.

The silver source is preferably selected from metallic silver and silver compounds, and
the nitrogen containing compound most preferably itself constitutes a non glucogenic compatible solute.

The expression "compatible solute" as used in this context refers to its most broadly accepted sense of organic compounds which serve as cytoplasmic solutes to balance water relations for cells growing in environments of high salt or sugar.

The expression "glucogenic" as used in this context refers to compounds that can be converted into glucose through a metabolic pathway that results in the generation of glucose from non-carbohydrate carbon substrates.

Compatible solutes are organic molecules accumulated in high concentrations in the cytoplasm allowing adaptation of the cell to varying salt concentrations. They are uncharged, highly water soluble and have to maintain an osmotic equilibrium with the surrounding medium. Some of these compounds also show secondary functions such as support and protection of macromolecules (e.g. enzymes). The cells may accumulate these compounds from the surrounding medium or by synthesis. Small glucogenic molecules (glycerol, maltose, trehalose, sucrose, amino acids, etc.) may also function as compatible solutes. Beside these compounds, most compatible solutes are N-containing compounds or polyols.
N-containing compounds are reported to be generally more potent than others. Cells must be equipped with a transporter system to concentrate "cheap" compatible solute compounds from outside. Compatible solutes such as amino acids and saccharides may be used in glucogensis for ATP production. Higher polyamines are also reported to act as anabolic growth regulators in fruits and vegetables and are involved in shelf life.
Plants are, through their leaves, an enormous area to be colonised by microbes. This area is nevertheless non attractive for microbes due to continuous water stress conditions on the wax layer. Temperature changes and UV irradiation are also stress factors for microbes. Pathogenic microbes will try to enter the leaves for better conditions. Spores have to germinate before attacking the plant. Germination of spores is a strong energy dependent process and time consuming. It is therefore interesting to regularly spray plants with the combination peroxide/silver to kill vegetative microbes and inhibit sporulation.
We detected that the combination peroxide/silver was more potent than the combination peroxide/alcohol in inhibiting fungal infections. Tests with dual peroxide combinations and silver (persulfate/hydrogen peroxide/silver) also showed promising results on the inhibition of spore formation. Some peroxides are very corrosive and irritating such as peroxyacetic acid, decompose quickly at higher temperature and are suspected to be mutagenic in bioassays. It is therefore advisable to lower the concentration in duo formulations or to include a silver compound to maintain the activity.

Compatible solutes, other than urea, do not interact with macromolecules and they have little impact on cell functions. This is in contrast to inorganic ions which bind to and destabilize organic molecules such as proteins and nucleic acids at relatively high concentrations. Compatible solutes are neutral (or zwitter ionic) at physiological pH. Some bacterial Compatible solutes are anionic but complex with cations. Cells may use at the same time different compatible solutes. They are interchangeable. Trimethylglycine (TMG) may replace sorbitol or other polyols in eukaryotic or prokaryotic cells to restore viability under hypertonic conditions. The use of urea is excluded here because it may destabilize macromolecules. Unmethylated amino acids are also excluded, because they are reported to all react with silver ions and are mostly glucogenic or ketogenic.

Non glucogenic compounds do not realize energetic reserves in the cell. Most of these compounds also show anti-oxidative activity in plants or other biological activities different from their osmotic activity. Compatible solutes may also show different effect depending on the environmental conditions. Trehalose protects enzymes at high temperature but inhibits them at normal temperature after intracellular concentration. They may be harmful in the absence of a perturbant (temperature). It is therefore important not to use high compatible solute concentrations. They may be especially harmful if used where their non-osmotic properties are not needed such as anti-oxidant activity, redox balancing activity, calcium modulation, detoxification activity, counteracting enzymatic inhibition or a compensatory activity. This may be different for a microbe and host cell (plant, human or animal), Microbes on leafs, roots or skin are mostly under osmotic stress while cellular host cells or tissues are under other kinds of stress such as oxidative stress.
The accumulation of osmolytes in crops under drought conditions is often cited in literature. However, field studies examining the association of osmolytes and crop yields have shown no consistent benefit. Besides a speculated benefit of turgor maintenance or root development in order to reach water, no clear increase in crop yield was observed. The secondary activity of the osmolyte (anti-oxidant) results in a kind of water deficit tolerance in plants. Hyper accumulation of compatible solutes may even be a symptom of injury.

Similar effects are seen in microbes using non glucogenic compatible solutes: maintenance of turgor pressure but no clear stimulation of growth under osmotic stress conditions. Cells normally synthesize compatible solutes as reaction on an osmotic up chock but degrade the osmolytes following and osmotic down chock. The initial response is mostly quicker when extracellular compatible solutes are taken up or released to the medium via transport systems instead of de novo synthesis.

When we look carefully at the literature concerning the effect of compatible solutes on microbes or microbial growth we encounter a lot of contradictions. This is mostly due to the use of complex growth media in which already a lot of compatible solutes are incorporated. Simple molecules such as mono-and disaccharides, amino acids and polyols are present in most culture media and have not only a function as compatible solute but in the first place an energy generating function. They are glucogenic and generate ATP. They contribute not only to osmoregulation but also to ATP-dependent expulsion of peroxides and silver compounds and to the absorption or expulsion of non-glucogenic compatible solutes.
Strains of Staphilococcus aureus show a significantly increase in membrane fluidity during the growth phase but decreasing fluidity entering in the stationary phase and decreasing sensitivity to biocidals. A lot of microbes are able to accumulate N-methylated or polyamine compatible solutes from the extern am medium. Accumulation of anti-stress compatible solutes, as TMG is a better compatible solute than the polyols, reaches higher intracellular levels and is less energy demanding than the denovo synthesis of polyols and dipeptides. Also in fungi, the synthesis of polyols is energy consuming, especially in solid-state fermentation where the water activity is decreasing. Addition of glucogenic compatible solute will help these organisms to synthesize new compatible solutes and to sustain expulsion activity against peroxides and silver compounds. Most compatible solutes are widespread in kingdoms of the tree of life, some are restricted to a small numbers of organisms. TMG, polyamins and ectoine are able to restore normal turgor pressure in stress conditions in prokaryotic and eukaryotic cells.

Some glucogenic compatible solute may initiate microbial growth when the peroxide is exhausted. In most published in vitro experiments, microbial suspensions are used and not microbial biofilms which are omnipresent (process water piping systems, containers, on leaves and roots, on contaminated surfaces, etc...). Biofilm embedded microbes are using "quorum sensing" molecules to counteract as one unit the increasing concentrations of anti-microbial compounds as antibiotics, disinfectants or pesticides. They make "global" decisions to expulse such molecules

through the elaboration of a dense network in the biofilm and the formation of new enzymes. The biofilm becomes stronger and stronger and increases in mass. It could be interesting to mislead the internal microbes using non glucogenic compatible solutes combined together with the use of antimicrobial compounds. Microbes are able to concentrate these solutes and to restore their turgor, but in this situation they are not able to synthesize new matrix molecules or to perform ATP dependent expulsion processes. The small compatible solute olecules are neutral and penetrate the biofilm matrix. Host tissues under biofilm matrix (infected tissues) could also benefit from the use of such compatible solutes and better resist oxidative stress conditions. The normalization of turgor on the microbial cytoplasmic membrane increases the access of peroxides and silver compounds. We discovered that use of non glucogenic compatible solutes is beneficial for the anti-microbial activity of the peroxide-silver combination in biofilm treatment.
In a medium containing non glucogenic compatible solutes, peroxides are able to destroy the organic matrix into non harmful substances through oxidation. Silver compounds activate their anti-microbial activity which is desirable due to the quick consumption of the peroxides in the presence of organic material. We also detected that the use of chelators such as EDTA, polyacrylates and phophonates, decreased the activity of the peroxide-silver combination. This is opposite to the use of chelators in various publications and patents. Non glucogenic compatible solutes also show interesting secondary activities, different from ATP generation, besides their osmolytic activity. This activity is beneficial to the host tissue.

Without intending to link the invention to any specific explanation, it is supposed that the use of compatible solutes may be appropriate for different reasons:
A) Activation of the antimicrobial activity resulting from the normalization of the cytoplasmatic turgor
B) Decrease of the oxidative stress in biological host tissues
C) Compatible solutes are non toxic and disappear in the medium because most organisms in the environment are able to concentrate these molecules. Selected compatible solutes are taken up by many organisms and are able to replace internally produced solutes under stress conditions
D) Residual compatible solutes are taken up by symbiotic organisms.
E) External supplied compatible solutes inhibit synthesis of other microbial compatible solutes.

It has been found, in particular, that the use of non glucogenic compatible solutes does not stimulate microbial growth at low concentrations but on the contrary activate anti-microbial activity of the peroxide-silver combination. Glucogenic solutes are mostly represented by mono-and disaccharides and amino acids.
Plants under biotic and abiotic stress are more vulnerable to infection and show a decrease in growth and development and therefore need to elaborate higher compatible solute concentrations for the entire plant including the roots and surrounding symbiotic organisms. Trehalose, a non reducing disaccharide found in different organisms, is synthesized under such conditions as compatible solute, stabilizes enzymes and membranes and protects structures from desiccation. It is present in higher concentrations in plants with diseases or in colonized plants.
Compatible solutes are synthesized under stress conditions but are again destroyed under normal conditions. Osmolyte concentrations in plants can also be used as an indicator of finished germination (malt quality, preharvest sprouting in seeds, ect...). Oxidative stress due to present of iron and copper ions in water supply stimulates the production of polyamines in plants.

It was also found that compatible solutes interfere with the action of silver/peroxide on the pathogens. The silver/peroxide also shows an anti-oxidative activity on the plants. This new combination is also useful in animal farming (poultry, pigs, horses, etc.) resulting in a higher production, a better feed-conversion besides the lowering effect on microbial pressure resulting in animal diseases. The non glucogenic compatible solutes are used simultaneous on the pathogens and the host. The compatible solutes are used at limited concentrations not inducing microbial growth resulting in the exhausting of the peroxides due to the microbial formation of catalase and peroxidase. The presence of a silver compound enhances the activity of the peroxide and reacts with peroxide inactivating enzymes. The combinations of peroxide, silver compounds and compatible solutes, used at appropriate concentrations, are less harmful than most pesticides or biocides. The amount of residues on fruits and vegetables are neglectable. Especially children are susceptible to the harmful effects of pesticides, cleaning products and tension active compounds. Higher occurrence of cancer, bird defects, leukaemia were found in children with early exposure to pesticides. In general, pesticides, biocides, and tensio active compounds provoke oxidative stress in their host. It is therefore interesting to add bioactive compatible solutes (non glucogenic at low concentrations) to the microbes normalizing turgor pressure and thereby increasing the target surface for the biocides. At the same time, a positive action is noticed on the host tissues, lowering the stress or consequences of inflammation or tissue damage.

The new antimicrobial compositions according to the invention can be diluted in all kinds of water and such a dilution is useful as disinfectant, antimicrobial biocide, hygienic compound, pesticide (algaecide, bactericide, fungicide, nematocide and virucide), decontamination compound or as anti-biofouling compound.

The composition is ecologically friendly and pharmaceutical preparations can also be used for topical applications in humans and animals.

According to a further preferred feature of the invention, the silver source of the a antimicrobial composition is most appropriately selected from
a) metallic silver, colloidal silver or silver nanoparticles
b) silver chloride nano particles or silver chloride nano particles made in situ on a natural carrier containing chloride ions or through addition of chloride ions, colloidal silver salt/carrier, silver oxide/carrier or ionic siver/carrier (carriers are low in thiols) or mixtures thereof,
c) silver nitrate, silver fluoride, silver chloride, silver oxide, silver zeolite, silver sodium hydrogen zirconium phosphate, silver carbonate, silver sulphide, silver selenate, silver sulphate or mixtures thereof.
d) silver amino acids complexes
e) silver iodide, silver chlorate, silver chromate, silver hydroxide, silver iodate, silver molybdate, silver oxalate, silver perchlorate, silver sulfardiazine, mild silver protein, silver thiosulfate or mixtures thereof.
f) silver salts of carboxylic acids and dicarboxylic acids or mixtures thereof, or mixtures of any of these

The non glucogenic compatible solute of the antimicrobial compositions according to the invention should not neutralize the activity of the silver compound and the concentration should be limited and linked to the silver concentration.

According to still further preferred features of the invention, the N-methylated aminoacids referred to above are preferably selected from glycine betaine (trimethyl glycine = TMG), dimethylglycine, sarcosine, carnitine, N-methyl alanine, taurine and trimethylamino-butyric acid, butyrobetaine, proline betaine (and other amino acid betaines);
the N-methylated aminoalcohol is preferably choline and choline derivatives such as choline-o-fosfate,glycerophosphorylcholine (GPC); the N-methylated amine oxide is preferably trimethylamine-oxide (TMAO); the N-methylated amidines are preferably selected from ectoine and hydroxyectoine; and
the poly amino hydrocarbon compounds are preferably selected from putrescine, cadaverine, spermine and spermidine" as well as polyamine growth factors from eucaryotic cells; and mixtures of any of these.

It is observed that suitable synergistic compounds in accordance with the invention, for supplementing the known antimicrobial effect of Silver + Peroxide thus include "betaine" derivatives.
It is specifically acknowledged in this context that it has been proposed in the state of the art (for instance in CN 1729787, GB 2 354 771, EP 1 225 887, EP 1 036 511, and in cosmetic formulations), to associate silver and peroxides with specific tension active "betaines", as surfactants.
It has to be stressed that the expression "betaines" in the more broad sense as used in the latter case, is totally different from the expression "betaine", stricto sensu, as used in respect of N-methylated aminoacids according to the invention. The N-methylated aminoacid, betaine type derivatives, according to the invention act as compatible solutes and not as surfactants.

Further preferred features of the antimicrobial composition of the invention involve that Further preferred features of the antimicrobial composition of the invention involve that
- the concentration (w/v) of the nitrogen containing compound or the compatible solute is lower than the peroxide concentration and not more than 100 times higher than the silver concentration;
- the total peroxide concentration ranges from 60% to 0.1% peroxide (in the concentrated solution);
- the organic peroxide is selected from a hydroperoxide, a peroxyacid, a perester type, a peroxy-methoxy acid or a peroxy-phenylacid, or combinations thereof, whereas the peroxyacid is preferably a peroxycarboxylic acid, a peroxydicarboxylic acid, a peroxytricarboxylic acid, a peroxy hydroxy acid or esters thereof and combinations thereof;
   the peroxyacid is peroxy lactic acid, peroxypropionic acid, peroxy citric acid, peroxyacetic acid, performic acid, perbenzoic acid, ethaneperoxoic acid, peroxy methoxyacetic acid, peroxyphenyl acetic acid, mono or diperoxydicaboxylic acid, mono or di-esters thereof and combinations thereof,
   the peroxycarboxylic acid is obtained through oxidation, with hydrogen peroxide or a hydroperoxide, of lactic acid, propionic acid, citric acid, oxalic acid, acetic acid, formic acid, benzoic acid, malonic acid, glutaric acid, pimelic acid, suberic acid, succinic acid, azeleic acid, adipic acid, sebasic acid, or derivatives thereof and esters thereof;
- the inorganic peroxide is an inorganic peroxyacid or a salt thereof, whereas
   the peroxyacid is:
   a) peroxymonosulfuric acid or peroxy disulfuric acid, sodium, potassium or ammonium salts thereof and mixtures thereof.
   b) peroxynitrous acid and salts thereof
   c) pernitrate and salts thereof
   d) urea peroxide or urea hydrogen peroxide
   e) percarbonate (example: sodiumpercarbonate)
   f) calcium, sodium, barium or magnesium peroxides and mixtures thereof
   g) hydrogen peroxide or activated hydrogen peroxide (hydrogen peroxide complexes of inorganic salts)
   h) permanganate
   i) perboric acid and salts thereof (example: sodium perborate), or mixtures of any of these
- the concentration of the inorganic peroxide varies between 50% and 0.1% (w/v) peroxide (in the concentrated composition);
- the silver concentration ranges from 10 mg/liter and 1000 mg/liter (in the concentrated composition);

The invention also specifically relates to the use of an antimicrobial composition as defined here above, as a disinfectant, antimicrobial biocide, hygienic compound, algaecide, bactericide, antimicrobial pesticide, fungicide, nematocide, virucide, decontaminating or anti-biofouling compound in a ready to use formulation or a concentrated formulation to be diluted in water or an aqueous solution, emulsion or suspension.

The invention also relates to the use of such antimicrobial composition as a topical composition concentrated or after dilution or as ingredient in the preparation of ointments, gels, crèmes, or any acceptable topical pharmaceutical preparation.

The invention also relates to such antimicrobial compositions comprising one or more additional fungal spore inhibiting compounds such as polyols, terpenes, carboxylic acid, sugar acids, boric acids, metal salts and combination thereof.

Further features and details of the invention will appear from the following specific, non limited examples of antimicrobial compositions and antimicrobial applications according to the invention.

### EXAMPLES of antimicrobial compositions

Example 1:
   H202: 50%
   AgNO3: 350 mg Ag/litre
   TMG: 0.05% (w/v)
Example 2:
   H202: 38-40%
   Colloidal Silver/carrier containing 350 mg Ag /litre (low thiol carrier) TMG: 0.1% (w/v)
Example 3:
   H202:50%
   AgNO3: 250 mg Ag/litre
   TMG: 0.2% (w/v)
Example 4:
   H202:40%
   AgNO3: 350 mg Ag/litre
   TMAO: 0.1% (w/v)
Example 5:
   H202:40%
   AgNO3: 400 mg Ag/litre
   Taurine: 0.3% (w/v)
Example 6:
   H202: 50%
   AgNO3: 300 mgAg/litre
   Carnitine: 0.25% (w/v)
Example 7:
H202:40%
   Silver(II)oxide (AgO) as colloid: 250 mg Ag/litre
   Potassium monopersulfate: 5%
   TMG: 0.5% (w/v)
Example 8:
   H202_{:}40% acidified with 0.1 N HCl
   AgCl (450 mg Ag /litre) made in situ on a natural carrier TMG: 2.5% (w/v)
Example 9:
   H202: 40%
   Silverthiosulfate (350 mg Ag /litre)
   TMAO: 0.1% (w/v)
Example 10:
   H202:40%
   Peracetic acid: 1.5%
   Silver nitrate: 300 mg Ag /litre
   TMG: 0.2%
Example 11:
   H202:40%
   Peracetic acid: 1.0%
   Silver nitrate: 360 mg Ag/litre
   PEG400:10%
   TMAO: 0.2%
Example 12:
   H202:50%
   Silver nitrate: 200 mg Ag /litre
   TMG: 0.05%

This solution can be used (as spray) after dilution in purified water for decontamination and disinfection (nano or micro droplets)
Example 13:
   H202:40%
   Potassium monopersulphate: 10%
   Silver nitrate: 250 mg Ag /litre
   PEG400: 10%
   TMG: 0;1%
Example 14:
   H202: 50%
   Silver chloride made in situ on a gum carrier: 380 mg Ag /litre
   TMG: 1%
Example 15:
   H202:50%
   Silver chloride made in situ on a thiol low natural carrier: 340 mg Ag /litre
   GPC: 0.05%
Examples 6:
   H202:50%
   Colloidal Silver/carrier containing 325 mg Ag /litre (carrier must be thiol low)
   TMG: 1%
Example 17:
   H202: 7.9%
   Colloidal Silver/carrier containing 70 mg Ag /litre (carrier must be thiol low)
   TMG: 0.1%

All antimicrobial compositions of the above examples, containing the mentioned peroxides, have been used as solution diluted 200 times (70 times in the case of Example 17) in source water (containing 150 mg/l chlorides), and have resulted in at least a 5 log titer reduction of Staphylococcus aureus at 25 degrees Celsius after 2 hours incubation.
Example 18:
   Calcium peroxide: 35%
   Silveroxide: 0.2% Silver
   TMG: 3% (w/v)
   Phosphate buffer: 20%
   Sodium percarbonate: 10%
   Silica until 100%

Prepare a 2% solution of this mixture before use for soil treatment or on crops. First dilute in demineralised water.
Example 19:
   Propylene glycol: 55%
   Sodium perborate monohydrate: 25%
   Silve nitrate: 0.3% Silver
   TMG: 3%
   Add fumed silica until 100%
   Dilute in water just before use.

Inorganic peroxides may be used in aquaculture to prevent growth of anaerobes and other bacteria; in poultry for decontamination of fodder, productivity increase and the improvement of egg quality; in cattle: inhibition of diarrhoea in calves and as anti-microbial compound, normalizing alimentary track and digestion.
For dental use: teeth bleaching and as antimicrobial compound
Example 20:
   45% H₂0₂
   5% polyol
   550 mg Ag/litre as AgNO₃
   0.2% TMG
Example 21:
   45% H₂0₂
   2.5% polyols
   0.3% malic acid
   0.15% peracetic acid
   580 mg Ag/litre as AgNO₃
   0.3% TMG
Example 22:
   45% H₂0₂
   2.5% polyol
   2% boric acid
   560 mg Ag/litre as AgNO₃
   0.3% menthol or eugenol
   0.3% TMG
Example 23:
   45% H₂0₂
   3% boric acid
   1 % galacturonic acid
   550 mg Ag/litre as AgNO₃
   1% sorbose
   0.3% TMG

### EXAMPLES of antimicobial APPICATIONS

### Example 24

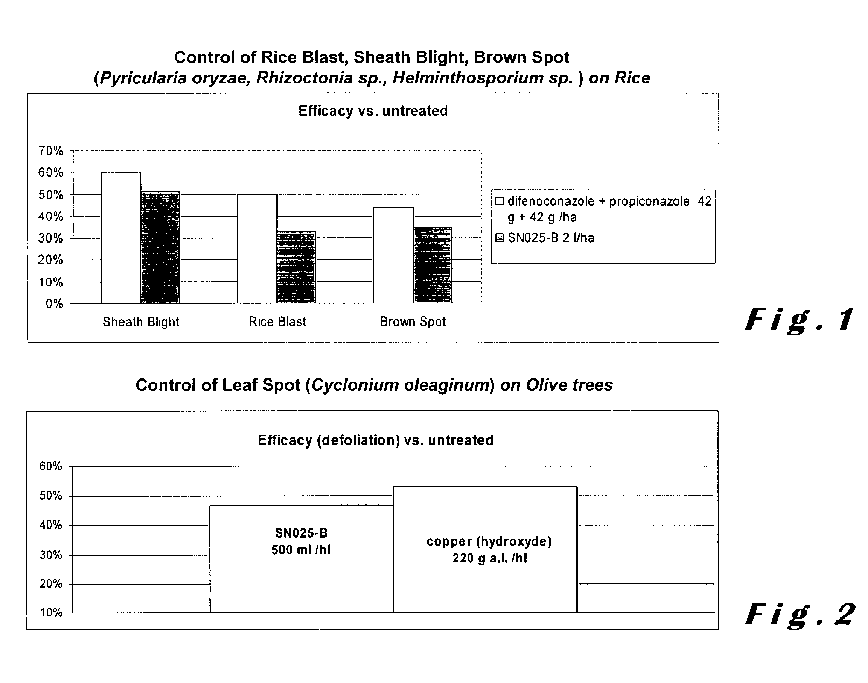

The antimicrobial composition of example 2 (composition labelled "SN025-B") was applied on rice for the control of Rice Blast, Sheath Blight, Brown Spot (Pyricularia oryzae, Rhizoctonia sp., Helminthosporium sp.)
Measurements were made for an average of two trials.
3 applications at 14 days interval
Assessments in Dec 2007 45-50 days sowing.
The results are illustrated in diagram 1 :
Strong infection in the untreated (64%, 52% & 50% for Rice Blast, Sheath Blight & Brown Spot respectively).
The composition SN025-B achieved 33-55% efficacy
(44-60% for a reference program with 2 triazoles)

### Example 25

The same composition labelled "SN025-B" was applied for the control of Leaf Spot (Cyclonium oleaginum) on Olive trees
Assessments on 8 May 2008 (% of defoliation on 200 leaves)
Applications in preventive spray schedule (2 application in Autumn and 2 applications in Spring)
The results are illustrated in diagram 2 :
SN025-B achieved 47% efficacy (comparable to Copper)

### Example 26

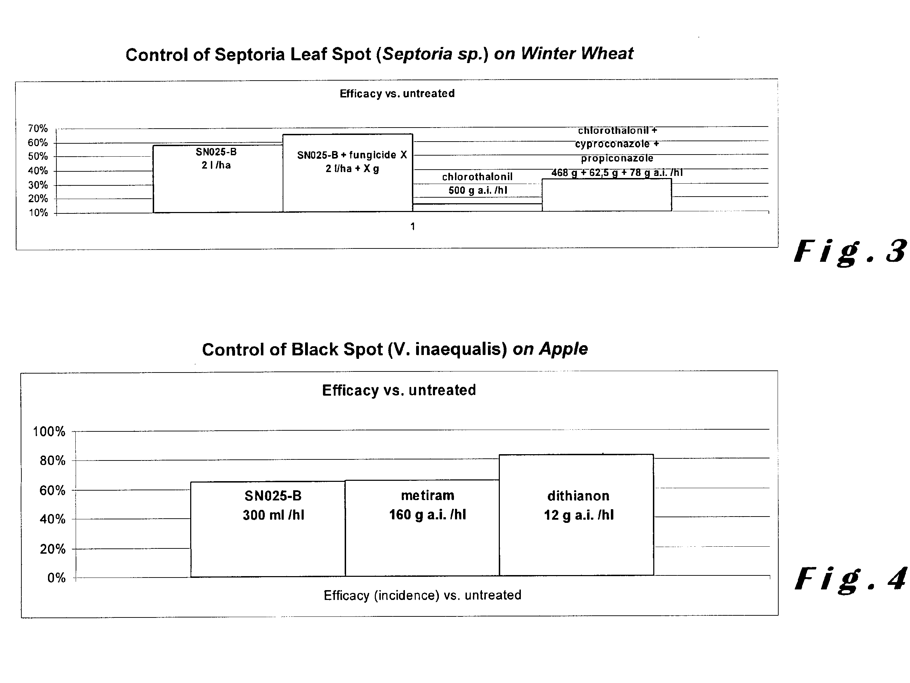

The same composition labelled "SN025-B" was applied for the control of Septoria Leaf Spot (Septoria sp.) on Winter Wheat
Assessments on 2 May 2008 (% of Septoria on L4)
One application at T0 (7 April 2008)
The results are illustrated in diagram 3 :
SN025-B achieved 58% efficacy

### Example 27

The same composition labelled "SN025-B" was applied for the control of Black Spot (V. inaequalis) on Apple
Assessments on 14 Dec 2007 and 3 Feb 2008
Applications in preventive spray schedule (at 7-14 days interval during primary contaminations, then at 14-28 days)
The results are illustrated in diagram 4 :
SN025-B achieved 65% efficacy (comparable to organic fungicides)

### Example 28

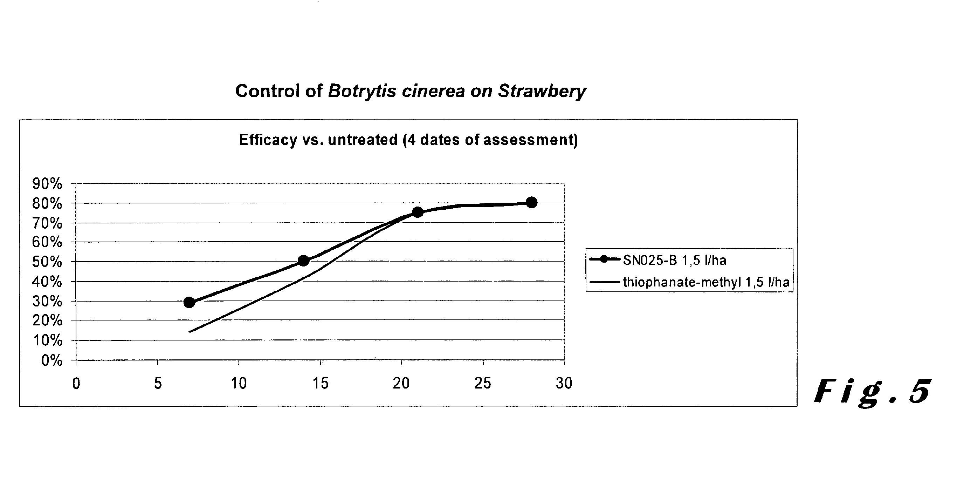

The same composition labelled "SN025-B" was applied for the control of Botrytis cinerea on Strawbery
Assessments at 7 days interval, starting 7 days after last application (4 applications in total)
The results are illustrated in diagram 5 :
SN025-B achieved 80% efficacy (comparable to organic fungicides)
Yield was 18.8% higher versus untreated and 7.9% higher versus thiophanate-methyl treated plots

## Claims

1. Antimicrobial composition comprising a combination of
- at least one organic and / or inorganic peroxide,
- at least one silver source, and
- at least one nitrogen containing compound selected from N-methylated aminoacids, N-methylated aminoalcohols, N-methylated amine oxides, N-methylated amidines and poly amino hydrocarbon compounds.

2. Antimicrobial composition according to claim 1, comprising a silver source selected from
a) metallic silver, colloidal silver or silver nanoparticles
b) silver chloride nano particles or silver chloride nano particles made in situ on a natural carrier containing chloride ions or through addition of chloride ions, colloidal silver salt/carrier, silver oxide/carrier or ionic siver/carrier or mixtures thereof,
c) silver nitrate, silver fluoride, silver chloride, silver oxide, silver zeolite, silver sodium hydrogen zirconium phosphate, silver carbonate, silver sulphide, silver selenate, silver sulphate or mixtures thereof.
d) silver amino acids complexes
e) silver iodide, silver chlorate, silver chromate, silver hydroxide, silver iodate, silver molybdate, silver oxalate, silver perchlorate, silver sulfardiazine, mild silver protein, silver thiosulfate or mixtures thereof.
f) silver salts of carboxylic acids and dicarboxylic acids or mixtures thereof,
or mixtures of any of these

3. Antimicrobial composition according to any one of claims 1 and 2, wherein the nitrogen containing compound is a non glucogenic compatible solute.

4. Antimicrobial composition comprising a combination of
- at least one organic and / or inorganic peroxide,
- at least one silver source, and
- at least one non glucogenic compatible solute.

5. Antimicrobial composition according to any one of claims 1 - 3, wherein the N-methylated aminoacid is selected from glycine betaine, dimethylglycine, sarcosine, carnitine, N-methyl alanine, taurine, trimethylamino-butyric acid, butyrobetaine and proline betaine,
the N-methylated aminoalcohol is selected from choline and choline derivatives,
the N-methylated amine oxide is trimethylamine-oxide (TMAO)
the N-methylated amidine is selected from ectoine, hydroxyectoine, and
the poly amino hydrocarbon compound is selected from putrescine, cadaverine, spermine and spermidine.

6. Antimicrobial composition according to any one of the preceding claims, wherein the concentration (w/v) of the nitrogen containing compound or the compatible solute is lower than the peroxide concentration and not more than 100 times higher than the silver concentration.

7. Antimicrobial composition according to any one of the preceding claims, wherein the total peroxide concentration ranges from 60% to 0.1% peroxide.

8. Antimicrobial composition according to any one of the preceding claims, wherein the organic peroxide is a hydroperoxide, a peroxyacid, a perester type, a peroxy-methoxy acid or a peroxyphenylacid, or combinations thereof.

9. Antimicrobial composition according to claim 8, wherein the peroxyacid is a peroxycarboxylic acid, preferably peroxy lactic acid, peroxypropionic acid, peroxy citric acid, peroxyacetic acid, performic acid, perbenzoic acid, ethaneperoxoic acid, peroxy methoxyacetic acid, or peroxyphenyl acetic acid; a mono or di peroxydicarboxylic acid; a peroxytricarboxylic acid; a peroxy hydroxy acid; or esters thereof and combinations thereof, whereas the peroxycarboxylic acid is preferably obtained through oxidation, with hydrogen peroxide or a hydroperoxide, of lactic acid, propionic acid, citric acid, oxalic acid, acetic acid, formic acid, benzoic acid, malonic acid, glutaric acid, pimelic acid, suberic acid, succinic acid, azeleic acid, adipic acid, sebasic acid, or derivatives thereof and esters thereof, .

10. Antimicrobial composition according to any one claims 1 - 7, wherein the inorganic peroxide is an inorganic peroxyacid or a salt thereof.

11. Antimicrobial composition according to any claim 10, wherein the peroxyacid is:
a) peroxymonosulfuric acid or peroxy disulfuric acid, sodium, potassium or ammonium salts thereof and mixtures thereof.
b) peroxynitrous acid and salts thereof
c) pernitrate and salts thereof
d) urea peroxide or urea hydrogen peroxide
e) percarbonate
f) calcium, sodium, barium or magnesium peroxides and mixtures thereof
g) hydrogen peroxide or activated hydrogen peroxide
h) permanganate
i) perboric acid and salts thereof,
or mixtures of any of these

12. Antimicrobial composition according to any one of the preceding claims, wherein the silver concentration ranges from 10 mg/liter and 1000 mg/liter.

13. Antimicrobial composition according to any one of the preceding claims, used as a disinfectant, antimicrobial biocide, hygienic compound, algaecide, bactericide, antimicrobial pesticide, fungicide, nematocide, virucide, decontaminating or anti-biofouling compound in a ready to use formulation or a concentrated formulation to be diluted in water or an aqueous solution, emulsion or suspension.

14. Antimicrobial composition according to any one of the preceding claims, used as a topical composition concentrated or after dilution or as ingredient in the preparation of ointments, gels, crèmes, or any acceptable topical pharmaceutical preparation.

15. Antimicrobial composition according to any one of the preceding claims, comprising one or more additional fungal spore inhibiting compounds such as polyols, terpenes, carboxylic acid, sugar acids, boric acids, metal salts and combination thereof

## Amended claims

### Amended claims in accordance with Rule 137(2) EPC.

**1.** Antimicrobial composition comprising a combination of
- at least one organic and / or inorganic peroxide,
- at least one silver source, and
- at least one nitrogen containing compound which is a non glucogenic compatible solute and which is selected from taurine,
choline and choline derivatives,
trimethylamine-oxide (TMAO),
ectoine and hydroxyectoine,
the N-methylated aminoacids glycine betaine, dimethylglycine,
sarcosine, carnitine, N-methyl alanine, trimethylamino-butyric acid, butyrobetaine and proline betaine, and
the poly amino hydrocarbon compounds putrescine, cadaverine, spermine and spermidine.

**2.** Antimicrobial composition according to claim 1, comprising a silver source selected from
a) metallic silver, colloidal silver or silver nanoparticles
b) silver chloride nano particles or silver chloride nano particles made in situ on a natural carrier containing chloride ions or through addition of chloride ions, colloidal silver salt/carrier, silver oxide/carrier or ionic siver/carrier or mixtures thereof,
c) silver nitrate, silver fluoride, silver chloride, silver oxide, silver zeolite, silver sodium hydrogen zirconium phosphate, silver carbonate, silver sulphide, silver selenate, silver sulphate or mixtures thereof.
d) silver amino acids complexes
e) silver iodide, silver chlorate, silver chromate, silver hydroxide, silver iodate, silver molybdate, silver oxalate, silver perchlorate, silver sulfardiazine, mild silver protein, silver thiosulfate or mixtures thereof.
f) silver salts of carboxylic acids and dicarboxylic acids or mixtures thereof,
or mixtures of any of these

**3.** Antimicrobial composition according to any one of the preceding claims, wherein the concentration (w/v) of the nitrogen containing compound is lower than the peroxide concentration and not more than 100 times higher than the silver concentration.

**4.** Antimicrobial composition according to any one of the preceding claims, wherein the total peroxide concentration ranges from 60% to 0.1% peroxide.

**5.** Antimicrobial composition according to any one of the preceding claims, wherein the organic peroxide is a hydroperoxide, a peroxyacid, a perester type, a peroxy-methoxy acid or a peroxy-phenylacid, or combinations thereof.

**6.** Antimicrobial composition according to claim 5, wherein the peroxyacid is a peroxycarboxylic acid, preferably peroxy lactic acid, peroxypropionic acid, peroxy citric acid, peroxyacetic acid, performic acid, perbenzoic acid, ethaneperoxoic acid, peroxy methoxyacetic acid, or peroxyphenyl acetic acid; a mono or di peroxydicarboxylic acid; a peroxytricarboxylic acid; a peroxy hydroxy acid; or esters thereof and combinations thereof, whereas the peroxycarboxylic acid is preferably obtained through oxidation, with hydrogen peroxide or a hydroperoxide, of lactic acid, propionic acid, citric acid, oxalic acid, acetic acid, formic acid, benzoic acid, malonic acid, glutaric acid, pimelic acid, suberic acid, succinic acid, azeleic acid, adipic acid, sebasic acid, or derivatives thereof and esters thereof,

**7.** Antimicrobial composition according to any one claims 1 - 4, wherein the inorganic peroxide is a weak or strong inorganic peroxyacid or a salt thereof.

**8.** Antimicrobial composition according to any claim 7, wherein the peroxyacid is:
a) peroxymonosulfuric acid or peroxy disulfuric acid, sodium, potassium or ammonium salts thereof and mixtures thereof.
b) peroxynitrous acid and salts thereof
c) pernitrate and salts thereof
d) urea peroxide or urea hydrogen peroxide
e) percarbonate
f) hydrogen peroxide or activated hydrogen peroxide
g) permanganate
h) perboric acid and salts thereof,
or mixtures of any of these

**9.** Antimicrobial composition according to any one of the preceding claims, wherein the silver concentration ranges from 10 mg/liter and 1000 mg/liter.

**10.** Antimicrobial composition according to any one of the preceding claims, used as a disinfectant, antimicrobial biocide, hygienic compound, algaecide, bactericide, antimicrobial pesticide, fungicide, nematocide, virucide, decontaminating or anti-biofouling compound in a ready to use formulation or a concentrated formulation to be diluted in water or an aqueous solution, emulsion or suspension.

**11.** Antimicrobial composition according to any one of the preceding claims, used as a topical composition concentrated or after dilution or as ingredient in the preparation of ointments, gels, crèmes, or any acceptable topical pharmaceutical preparation.

**12.** Antimicrobial composition according to any one of the preceding claims, comprising one or more additional fungal spore inhibiting compounds such as polyols, terpenes, carboxylic acid, sugar acids, boric acids, metal salts and combination thereof
